# EUROPEAN PATENT APPLICATION

(11) **EP 4 647 006 A1**
(43) Date of publication of application: **12.11.2025**
(21) Application number: 24756754.8
(22) Date of filing: 06.02.2024
(51) Int. Cl.: A61B 5/16, A61B 5/00

(54) **LIFESTYLE IMPROVEMENT SYSTEM**

(30) Priority: 15.02.2023 JP 2023021847
(71) Applicant: NISHIKAWA CO., LTD., Tokyo 103-0006 (JP)
(72) Inventor: NISHIKAWA, Yasuyuki, Tokyo 103-0006 (JP); NONOMURA, Takuto, Tokyo 103-0006 (JP); SHIMADA, Saki, Tokyo 103-0006 (JP); NISHINA, Midori, Tokyo 103-0006 (JP); AOKI, Mari, Tokyo 103-0006 (JP)
(74) Representative: Viering, Jentschura & Partner mbB Patent- und Rechtsanwälte
(86) International application number: PCT/JP2024/003956
(87) International publication number: WO 2024/171906

(57) **Abstract**

A lifestyle improvement system includes a sensor unit that acquires respiratory information, body movement information, and heartbeat information; a sleep state acquisition unit that acquires sleep state information from at least one of the respiratory information, the body movement information, and the heartbeat information; an autonomic nervous system acquisition unit that acquires autonomic nervous system information from the heartbeat information; a device control unit that controls an operation of a device based on at least one of past sleep information stored in advance and the autonomic nervous system information; a prediction information generation unit that generates prediction information from at least one of the past sleep information and the autonomic nervous system information; and an improvement information generation unit that generates improvement information from the prediction information.

## Description

### Technical Field

The present disclosure relates to a lifestyle improvement system.

Priority is claimed on Japanese Patent Application No. 2023-021847, filed on February 15, 2023, the entire content of which is incorporated herein by reference.

### Background Art

Japanese Unexamined Patent Publication No. 2006-87850 describes a sleep state detection system that detects the sleep state of a human. The sleep state detection system includes a radio wave transmitting and receiving device that emits microwaves toward a human body during sleep and receives reflected waves of the microwaves affected by the body pulsations of the human body, and a computer that detects the sleep state of a human by analyzing the reflected wave data of the reflected waves. The computer performs operation and stop control of an air conditioning device, a lighting device, and an audio device based on the detected sleep state.

### Citation List

### Patent Literature

Patent Literature 1: Japanese Unexamined Patent Publication No. 2006-87850

### Summary of Invention

### Technical Problem

The sleep state detection system described above performs operation and stop control of the air conditioning device, the lighting device, and the audio device in accordance with the sleep state of a user from the time of going to bed to waking up. However, in order to improve the lifestyle of the user, it may be required to predict the state of the user while awake after waking up and to provide information for improving the lifestyle of the user.

An object of the present disclosure is to provide a lifestyle improvement system capable of predicting the state of a user while awake and providing information for improving the lifestyle of the user.

### Solution to Problem

A lifestyle improvement system according to one aspect of the present disclosure is (1) a lifestyle improvement system for improving a lifestyle of a user. The lifestyle improvement system includes a sensor unit that acquires at least one of respiratory information that is information indicating a respiration of the user, body movement information that is information indicating a body movement of the user, and heartbeat information that is information indicating a heartbeat of the user; a sleep state acquisition unit that acquires sleep state information, which is information indicating a sleep state of the user, from at least one of the respiration information, the body movement information, and the heartbeat information; an autonomic nervous system acquisition unit that acquires autonomic nervous system information, which is information indicating a state of an autonomic nervous system of the user, from the heartbeat information; a device control unit that controls an operation of a device constituting an environment surrounding the user, based on at least one of past sleep information, which is past sleep state information of the user stored in advance, and the autonomic nervous system information; a prediction information generation unit that generates prediction information, which is information indicating a predicted state of the user while awake, from at least one of the past sleep information and the autonomic nervous system information; and an improvement information generation unit that generates improvement information, which is information for improving the lifestyle of the user, from the prediction information.

The lifestyle improvement system generates the prediction information of the user from at least one of the past sleep information and the autonomic nervous system information. The lifestyle improvement system can provide, for example, the user with information indicating the mental and physical state of the user as the prediction information. The lifestyle improvement system generates the improvement information from the prediction information. For example, when the mental and physical state of the user is predicted to tend to become poor, the lifestyle improvement system can provide the user with information indicating that the user is recommended not to make an important decision as the improvement information. The lifestyle improvement system not only can control the device while the user sleeps, but can also predict the state of the user while awake based on the sleep of the user and provide information for improving the lifestyle of the user based on the sleep state. In the lifestyle improvement system, the prediction information and the improvement information can be generated from the past sleep state of the user. The prediction information indicating the predicted state of the user while awake and the improvement information for improving the lifestyle can be generated by taking into account not only the sleep state of that day but also the past sleep state of the user. Compared to when the prediction information and the improvement information are generated only from the sleep state of the user from the time of going to bed to waking up, the prediction information and the improvement information can be generated based on longer-term records. As a result, the prediction information and the improvement information can be generated more accurately.

(2) In (1) above, the sensor unit may include a cushion sensor attached to a cushion on which the user is seated. In this case, for example, vital data of the user can be acquired not only during sleep but also during the day. Since the prediction information and the improvement information can be generated by taking into account the vital data of the user during the day, the prediction information and the improvement information can be generated more accurately using the state of the user both when asleep and when awake.

(3) In (1) or (2) above, the prediction information may include mental information that is information indicating a mental state of the user, physical condition information that is information indicating a physical condition of the user, and brain information that is information indicating a state of a brain of the user. In this case, the mental, physical condition, and brain states of the user while awake can be predicted.

(4) In (3) above, the physical condition information may include skin information indicating a state of a skin of the user. In this case, the information indicating the state of the skin of the user can be provided as the prediction information.

(5) In any one of (1) to (4) above, the improvement information generation unit may generate the improvement information from subjective information that is information indicating a subjective evaluation of the user. In this case, since the improvement information can be generated by taking into account the subjective evaluation of the user, the improvement information can be generated more accurately.

(6) In any one of (1) to (5) above, the improvement information may include information indicating a type of clothing recommended for the user. In this case, the information indicating the type of clothing recommended for the user can be provided as the improvement information.

(7) In any one of (1) to (6) above, the improvement information may include information indicating a type of food recommended for the user. In this case, the information indicating the type of food recommended for the user can be provided as the improvement information.

(8) In any one of (1) to (7) above, the improvement information may include information indicating a type of cosmetics recommended for the user. In this case, the information indicating the type of cosmetics recommended for the user can be provided as the improvement information.

(9) In any one of (1) to (8) above, the device may include a pillow used by the user for sleeping, and the device control unit may control an angle of a placement surface of the pillow, on which a head of the user is placed, with respect to a horizontal plane. In this case, since the angle of the placement surface of the pillow can be adjusted while the user sleeps, the sleep state of the user can be further improved.

(10) In any one of (1) to (9) above, the lifestyle improvement system may further include an arrhythmia detection unit that detects a presence or absence of arrhythmia in the user from the heartbeat information acquired by the sensor unit. In this case, information on the presence or absence of arrhythmia while asleep or while awake can be acquired by detecting the presence or absence of arrhythmia from the heartbeat information acquired by the sensor unit. The user can identify the presence or absence of his or her own arrhythmia.

A lifestyle improvement system according to another aspect of the present disclosure is (11) a lifestyle improvement system for improving a lifestyle of a user. The lifestyle improvement system includes a sensor unit that acquires heartbeat information that is information indicating a heartbeat of the user; an autonomic nervous system acquisition unit that acquires autonomic nervous system information, which is information indicating a state of an autonomic nervous system of the user, from the heartbeat information; a prediction information generation unit that generates prediction information, which is information indicating a predicted state of the user while awake, from the autonomic nervous system information; and an improvement information generation unit that generates improvement information, which is information for improving the lifestyle of the user, from the prediction information.

The lifestyle improvement system generates the prediction information of the user from the autonomic nervous system information, and generates the improvement information from the prediction information. Therefore, similarly to the lifestyle improvement system described above, the state of the user while awake can be predicted, and the information for improving the lifestyle of the user can be provided.

### Advantageous Effects of Invention

According to the present disclosure, the state of the user while awake can be predicted, and the information for improving the lifestyle of the user can be provided.

### Brief Description of Drawings

[FIG. 1] FIG. 1 is a block diagram showing a lifestyle improvement system as one example.
[FIG. 2] FIG. 2 is a view showing an example of an input screen for subjective information displayed on an information terminal shown in FIG. 1.
[FIG. 3] (a) in FIG. 3 is a perspective view showing a cushion body to which a sensor sheet is attached. (b) in FIG. 3 is a perspective view showing a core material constituting the cushion body shown in (a) of FIG. 3.
[FIG. 4] FIG. 4 is a perspective view showing the core material of a cushion to which a cushion sensor is attached.
[FIG. 5] FIG. 5 is a view showing an example of an output screen for prediction information and improvement information displayed on the information terminal shown in FIG. 1.
[FIG. 6] FIG. 6 is a view showing an example of an output screen for feedback information displayed on the information terminal shown in FIG. 1.
[FIG. 7] FIG. 7 is a flowchart showing one example of the operation of the lifestyle improvement system.
[FIG. 8] FIG. 8 is a flowchart showing an example of the arrhythmia detection function of the lifestyle improvement system.

### Description of Embodiments

Hereinafter, an example of a lifestyle improvement system according to the present disclosure will be described with reference to the drawings. In the description of the drawings, the same or corresponding elements are denoted by the same reference signs, and duplicate descriptions will be omitted as appropriate. For ease of understanding, the drawings may be depicted in a partially simplified or exaggerated manner, and dimensional ratios and the like are not limited to those shown in the drawings.

The lifestyle improvement system according to the present disclosure improves the lifestyle of a user. For example, the lifestyle improvement system may be used for personal or household purposes, or may be used in research institutes or the like for testing and research purposes. The lifestyle improvement system may be used in hospitals or the like for treatment purposes. The term "user" refers to a person who is the target of lifestyle improvement by the lifestyle improvement system. The user is, for example, a person who has a health risk due to disruption in lifestyle or the like, a person who receives treatment at a hospital or the like, or a person who wishes to improve his or her own lifestyle.

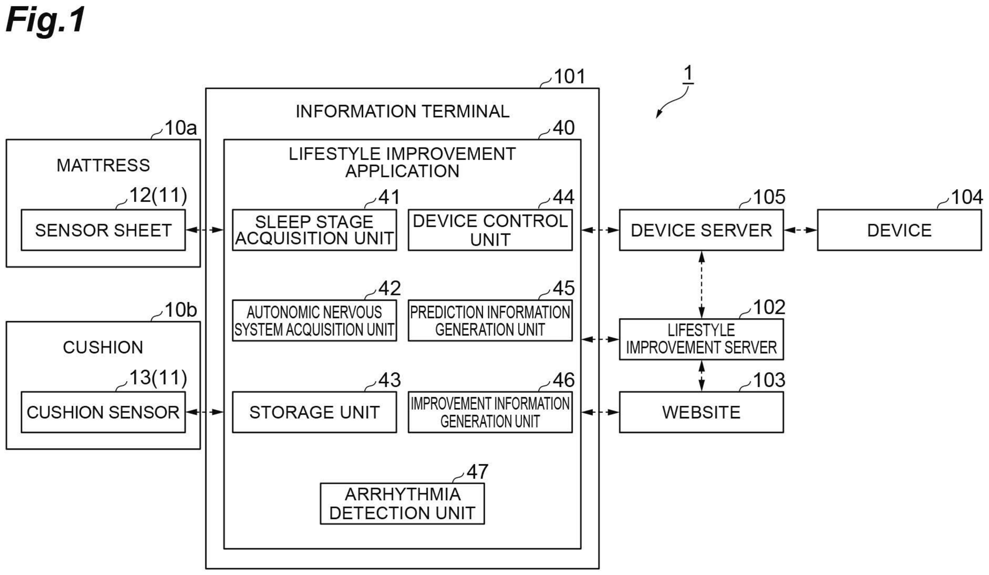

FIG. 1 is a block diagram showing a lifestyle improvement system 1 as one example. The lifestyle improvement system 1 includes a sensor unit 11, an information terminal 101, and a lifestyle improvement server 102.

The information terminal 101 is, for example, a mobile terminal. The "mobile terminal" is, for example, a portable information terminal such as a mobile phone including a smartphone, a tablet, a notebook computer, or a wearable terminal such as a wristwatch. The information terminal 101 may be a terminal other than a mobile terminal, or may be, for example, a desktop computer. The information terminal 101 includes, as one example, a processor (for example, a CPU) that executes an operating system, software (application), and the like; a main storage unit composed of a ROM and a RAM; an auxiliary storage unit composed of a flash memory and the like; a communication control unit composed of a wireless communication module and the like; an input device; and an output device such as a display. However, the configuration of the information terminal 101 is not limited to the above-described configuration, and can be changed as appropriate.

In the information terminal 101, a lifestyle improvement application 40 is executed as an application program. The lifestyle improvement application 40 may be an application downloaded to the information terminal 101 and executed on the information terminal 101, or may be executed on the lifestyle improvement server 102. The lifestyle improvement application 40 may be an application downloaded from the lifestyle improvement server 102. Hereinafter, an example in which the lifestyle improvement application 40 is an application downloaded to the information terminal 101 and the functions of the lifestyle improvement application 40 are executed on the information terminal 101 will be described.

Each function of the lifestyle improvement application 40 is realized by loading predetermined software into the processor or the main storage unit and executing the software. The processor operates the communication control unit, the input device, or the output device described above in accordance with the software, and reads and writes data from and to the main storage unit or the auxiliary storage unit. Data or databases required to execute the functions of the lifestyle improvement application 40 are stored in the main storage unit or the auxiliary storage unit.

Each functional element of the information terminal 101 is realized by loading predetermined software into the processor or the storage unit (for example, the main storage unit or the auxiliary storage unit described above) and executing the software. The processor operates the communication control unit, the input device, or the output device described above in accordance with the software, and reads and writes data from and to the storage unit. Data or databases used in the processing of the lifestyle improvement server 102 are stored in the storage unit.

The lifestyle improvement application 40 may be a distributed processing system composed of a plurality of computers, or may be a client-server system or a cloud system. The lifestyle improvement application 40 includes, for example, a main module, a data acquisition module, a determination module, and an output module. The data acquisition module, the determination module, and the output module are executed, thereby causing each functional element of the lifestyle improvement application 40 to function. As one example, the lifestyle improvement application 40 may be provided in a state in which the lifestyle improvement application 40 is fixedly recorded on a tangible storage medium such as a CD-ROM, a DVD-ROM, or a semiconductor memory. The lifestyle improvement application 40 may be provided via a communication network as a data signal superimposed on a carrier wave. A functional configuration of the lifestyle improvement application 40 will be described later.

The information terminal 101 is a terminal that allows information to be input by accepting an operation from the user. The information terminal 101 can transmit the input information to the lifestyle improvement server 102. For example, the information terminal 101 transmits subjective information, which is information indicating a subjective evaluation of the user, to the lifestyle improvement server 102.

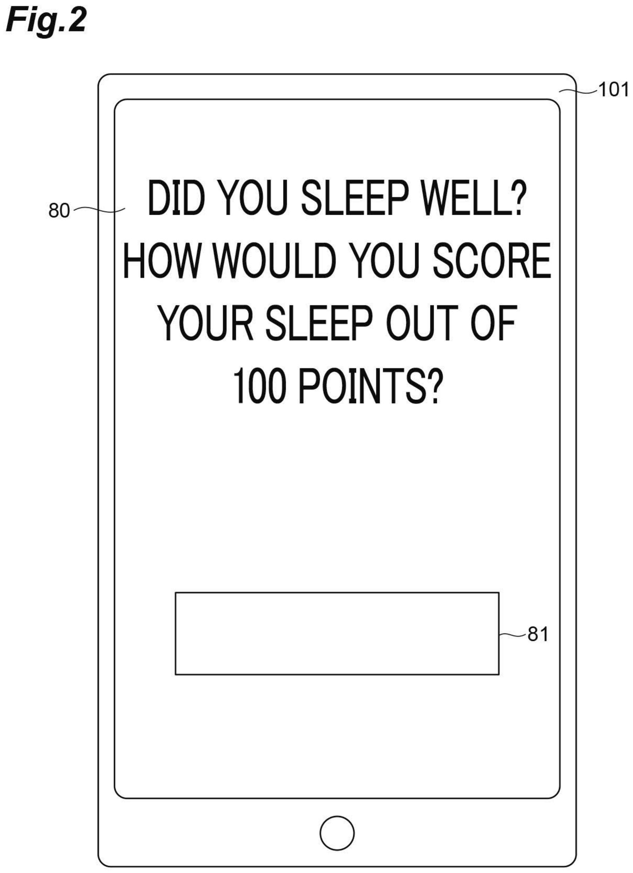

FIG. 2 is a view showing an example of an input screen 80 for the subjective information displayed on the information terminal 101. The input screen 80 is a screen to which the subjective information is input. The subjective information includes, for example, a subjective evaluation of sleep. The input screen 80 includes a sleep evaluation input portion 81 that is a portion to which the subjective evaluation of sleep is input. For example, the subjective evaluation of sleep is input to the sleep evaluation input portion 81 as a score with a maximum score of 100 points.

The information terminal 101 displays prediction information and improvement information. Details of the prediction information and the improvement information will be described later.

The information terminal 101 can access a predetermined website 103. For example, the website 103 includes at least one of an online shopping site that handles products including at least one of clothing, food, soap, and cosmetics, and a travel site for advertising travel and accepting reservations for accommodations. The website 103 transmits information to the information terminal 101 in response to a request from the information terminal 101. The information transmitted by the website 103 includes, for example, a Uniform Resource Locator (URL). The information transmitted from the website 103 to the information terminal 101 is provided to the user by being displayed on the information terminal 101. The website 103 may be accessible from the lifestyle improvement server 102.

The sensor unit 11 acquires information on the body of the user. The sensor unit 11 acquires at least one of respiratory information, body movement information, and heartbeat information. As one example, the sensor unit 11 acquires the respiratory information, the body movement information, and the heartbeat information. The respiratory information is information indicating the respiration of the user. The body movement information is information indicating the body movement of the user. The heartbeat information is information indicating the heartbeat of the user. The heartbeat information includes, for example, the waveform of the heartbeat. The contents of the information acquired by the sensor unit 11 can be changed as appropriate. The sensor unit 11 may further acquire at least one of information indicating the body temperature of the user, information indicating blood pressure, and information indicating blood glucose level. The sensor unit 11 includes a sensor sheet 12 attached to a mattress 10a, and a cushion sensor 13 attached to a cushion 10b.

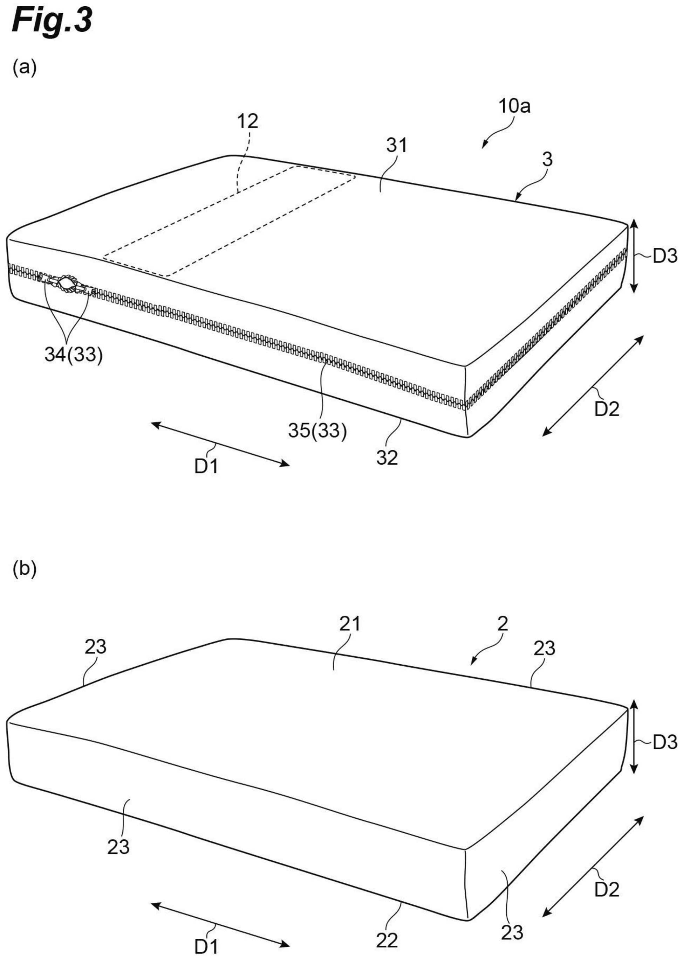

(a) in FIG. 3 is a perspective view showing the mattress 10a to which the sensor sheet 12 is attached. (b) in FIG. 3 is a perspective view showing a core material 2 constituting the mattress 10a shown in (a) of FIG. 3. For example, the sensor sheet 12 is attachable to and detachable from the mattress 10a. The sensor sheet 12 acquires vital data of the user lying on the mattress 10a. As one example, the sensor sheet 12 may acquire an electrocardiogram. The respiratory information, the body movement information, and the heartbeat information described above are included, for example, in the vital data. The sensor sheet 12 acquires the respiratory information, the body movement information, and the heartbeat information of the user during sleep.

For example, the sensor sheet 12 includes a sheet-shaped fabric on which a thread-shaped sensor is embroidered, and the position of the sheet-shaped fabric with respect to the mattress 10a can be changed. The thread-shaped sensor of the sensor sheet 12 is fixed, for example, by being embroidered so as to spread two-dimensionally on the sheet-shaped fabric. The thread-shaped sensor is, as one example, a piezoelectric sensor. In this case, the piezoelectric sensor of the sensor sheet 12 generates electrical signals corresponding to the applied load of the body of the user based on the load. The sensor sheet 12 acquires the electrical signals as the respiratory information, the body movement information, and the heartbeat information described above. The electrical signals acquired as the respiratory information, the body movement information, and the heartbeat information may be referred to as "signals" below.

The sensor sheet 12 includes, for example, the thread-shaped sensor (as one example, a piezoelectric sensor), and a communication unit that outputs the respiratory information, the body movement information, and the heartbeat information to the outside of the sensor sheet 12 as electrical signals generated by the thread-shaped sensor. An example in which the sensor sheet 12 includes a piezoelectric sensor has been described above. However, the type of sensor of the sensor sheet 12 is not limited to a piezoelectric sensor, and is not particularly limited. For example, the sensor sheet 12 may include an acceleration sensor.

The respiratory information includes a signal indicating body movement associated with the respiration of the user. The term "body movement associated with respiration" refers to the movement of the body associated with respiration. The sensor sheet 12 detects body movement associated with the respiration of the user. The sensor sheet 12 outputs a signal indicating the body movement associated with respiration to the outside of the sensor sheet 12. The heartbeat information includes a signal indicating body movement associated with the heartbeat of the user. The term "body movement associated with heartbeat" refers to the movement of the body associated with heartbeat. The sensor sheet 12 detects body movement associated with the heartbeat of the user. The sensor sheet 12 outputs a signal indicating the body movement associated with heartbeat to the outside of the sensor sheet 12. The body movement information includes a signal indicating body movement not associated with the respiration and heartbeat of the user. The term "body movement not associated with respiration and heartbeat" refers to the movement of the body that is not related to respiration and heartbeat, for example, the movement of the body caused by turning over during sleep. The sensor sheet 12 detects body movement not associated with the respiration and heartbeat of the user. The sensor sheet 12 outputs a signal indicating the body movement not associated with respiration and heartbeat to the outside of the sensor sheet 12.

For example, the sensor sheet 12 is used while attached to the mattress 10a on which the body of the user is placed. The mattress 10a has a rectangular shape in a plan view. The mattress 10a extends in a longitudinal direction D1 and a lateral direction D2 orthogonal to the longitudinal direction D1. The mattress 10a has a thickness in a thickness direction D3 orthogonal to both the longitudinal direction D1 and the lateral direction D2.

A length of the mattress 10a in the longitudinal direction D1 is, for example, 120 cm or more and 210 cm or less (as one example, 195 cm). A length of the mattress 10a in the lateral direction D2 is, for example, 70 cm or more and 180 cm or less (as one example, 97 cm). A length of the mattress 10a in the thickness direction D3 is, for example, 3 cm or more and 40 cm or less (as one example, 9 cm).

For example, the user of the mattress 10a places his or her own body on the mattress 10a. At this time, a direction in which the body of the lying user extends (namely, a direction in which the head and legs of the user are connected to each other) coincides with, for example, the longitudinal direction D1 of the mattress 10a.

As shown in (b) of FIG. 3, the mattress 10a includes the core material 2 housed inside a cover fabric 3 to be described later. For example, the core material 2 has a rectangular shape in a plan view. The core material 2 includes, for example, a content and a bag that houses the content. The content is, for example, urethane foam or polyester. The material of the bag is, for example, cotton or polyester.

The core material 2 has a rectangular parallelepiped shape. The core material 2 has an upper surface 21 on which the body of the user is placed, and a lower surface 22 facing opposite the upper surface 21. The upper surface 21 is a surface facing one side (the upper side in (b) of FIG. 3) in the thickness direction D3. The lower surface 22 is a surface facing the other side (the lower side in (b) of FIG. 3) in the thickness direction D3. The core material 2 has a plurality of side surfaces 23 connecting the upper surface 21 and the lower surface 22.

As shown in (a) of FIG. 3, the mattress 10a includes the cover fabric 3. For example, the cover fabric 3 has a bag shape, and has a rectangular shape in a plan view. The cover fabric 3 includes, for example, a front fabric 31 covering the upper surface 21 of the core material 2; a back fabric 32 covering the lower surface 22 of the core material 2; and an opening and closing member 33 connecting the front fabric 31 and the back fabric 32 to each other. The opening and closing member 33 is provided on the cover fabric 3 at a position corresponding to the side surfaces 23 of the core material 2. When viewed in the thickness direction D3, the opening and closing member 33 is located outside the side surfaces 23 of the core material 2. The cover fabric 3 is attachable to and detachable from the core material 2. The term "being attachable to and detachable from the core material" includes rolling up the cover fabric with respect to the core material, and rolling up the cover fabric to expose at least a part of the core material.

The opening and closing member 33 is, as one example, a so-called double slider. The opening and closing member 33 may include two slide members 34 and an opening and closing portion 35 that opens and closes the front fabric 31 and the back fabric 32 when the slide members 34 are slid.

For example, the front fabric 31 and the back fabric 32 can be opened by sliding the first slide member 34 along the opening and closing portion 35 in a first direction (a clockwise direction in (a) of FIG. 3) and sliding the second slide member 34 along the opening and closing portion 35 in a second direction (a counterclockwise direction in (a) of FIG. 3) opposite the first direction.

In addition, the front fabric 31 and the back fabric 32 can be closed by sliding the first slide member 34 along the opening and closing portion 35 in the second direction and sliding the second slide member 34 along the opening and closing portion 35 in the first direction. The opening and closing member 33 may be a so-called single slider. In this case, the opening and closing member 33 includes one slide member 34 and the opening and closing portion 35.

The sensor sheet 12 is disposed, for example, inside the cover fabric 3 having a bag shape. The sensor sheet 12 is disposed between the core material 2 and the cover fabric 3. When the sensor sheet 12 is attached to the mattress 10a, for example, the sensor sheet 12 is disposed to extend in the lateral direction D2 of the mattress 10a. In this case, the sensor sheet 12 is located on the side opposite the body of the user when viewed from the cover fabric 3 of the mattress 10a. Therefore, when the body of the user is placed on the mattress 10a, the sensor sheet 12 does not come into contact with the user. The sensor sheet 12 acquires the respiratory information, the body movement information, and the heartbeat information of the user in a non-contact manner.

The sensor sheet 12 is attached, for example, at a position corresponding to the heart of the user in the longitudinal direction D1. As one example, the sensor sheet 12 is attached at a position spaced apart in the longitudinal direction D1 of the mattress 10a from the position where the head of the user is placed (a position on the lower right side in (a) of FIG. 3). The attachment position of the sensor sheet 12 in the longitudinal direction D1 can be changed. A distance from the position where the head of the user is placed to the position where the sensor sheet 12 is attached may be, for example, 40 cm or more and 50 cm or less (as one example, 50 cm).

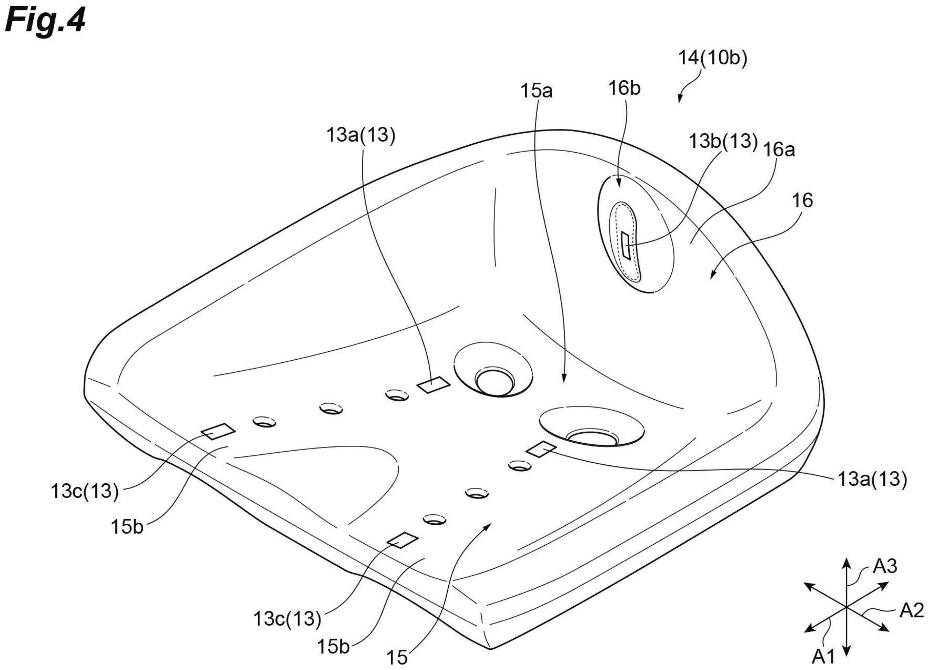

FIG. 4 is a perspective view showing a core material 14 of the cushion 10b to which the cushion sensor 13 is attached. The cushion 10b includes, for example, the core material 14 and a fabric (not shown) having a bag shape that houses the core material 14. The core material 14 includes a seating portion 15 extending in a horizontal direction, and a lumbar support portion 16 extending upward from the seating portion 15. The core material 14 is made of, for example, a flexible material such as urethane foam.

The seating portion 15 extends in a first direction A1 and a second direction A2 intersecting the first direction A1. The first direction A1 is a front-rear direction when viewed from the user seated on the seating portion 15, and the second direction A2 is a left-right direction when viewed from the user seated on the seating portion 15. The seating portion 15 has a thickness in a third direction A3 intersecting both the first direction A1 and the second direction A2. For example, the third direction A3 is a vertical direction. The seating portion 15 includes a buttock support portion 15a and two thigh support portions 15b. The buttock support portion 15a and the thigh support portions 15b are disposed to be aligned along the first direction A1.

Hereinafter, a forward direction when viewed from the user seated on the seating portion 15 may be referred to as "front", "front side", or "forward", and a direction opposite the forward direction may be referred to as "rear", "rear side", or "rearward". However, the directions are defined for convenience of description, and do not limit the position, orientation, and the like of each portion.

The buttock support portion 15a is located on the rear side of the seating portion 15. The buttocks of the user are placed on the buttock support portion 15a. The buttock support portion 15a supports the buttocks of the user. The two thigh support portions 15b are aligned along the second direction A2 on the front side of the seating portion 15. The back sides of the thighs of the user are placed on the thigh support portions 15b. The thigh support portions 15b support the back sides of the thighs of the user.

The lumbar support portion 16 extends upward from an end portion (rear end) of the seating portion 15 in the first direction A1. The lumbar support portion 16 includes, for example, a general portion 16a extending in both the second direction A2 and the third direction A3, and a sacrum support portion 16b located at the center of the general portion 16a in the second direction A2. The general portion 16a refers to a portion of the lumbar support portion 16 excluding the sacrum support portion 16b.

The sacrum support portion 16b has a convex shape protruding forward. The sacrum support portion 16b protrudes forward from the general portion 16a. As one example, the shape of the sacrum support portion 16b when viewed from the front is a shape having a major axis and a minor axis. For example, the shape of the sacrum support portion 16b when viewed from the front is an oval shape (as one example, an elliptical shape).

The cushion sensor 13 acquires vital data of the user seated on the cushion 10b. For example, the cushion sensor 13 acquires the heartbeat information of the user during the day. The cushion sensor 13 is, for example, a piezoelectric sensor fixed to the core material 14. In this case, the cushion sensor 13 measures pressure that is applied to each portion of the cushion 10b from the body of the user placed on the cushion 10b. The type of the cushion sensor 13 is not particularly limited. The cushion sensor 13 includes, for example, a seating portion sensor 13a attached to the seating portion 15; a sacrum support portion sensor 13b attached to the sacrum support portion 16b; and a thigh support portion sensor 13c attached to each of the two thigh support portions 15b.

The seating portion sensor 13a detects that the user is seated on the seating portion 15. For example, the seating portion sensor 13a detects that the ischium of the user is in contact with the seating portion sensor 13a. The sacrum support portion sensor 13b measures, for example, the load of the body of the user that comes into contact with the sacrum support portion sensor 13b. The thigh support portion sensor 13c measure, for example, the load of the thighs of the user seated on the seating portion 15. The seating portion sensor 13a, the sacrum support portion sensor 13b, and the thigh support portion sensors 13c generate an electrical signal corresponding to the load of the body of the user based on the load, and acquires, for example, the electrical signal as the heartbeat information described above.

The functional configuration of the lifestyle improvement application 40 will be described. As shown in FIG. 1, the lifestyle improvement application 40 includes, as the functional configuration, a sleep state acquisition unit 41, an autonomic nervous system acquisition unit 42, a storage unit 43, a device control unit 44, a prediction information generation unit 45, an improvement information generation unit 46, and an arrhythmia detection unit 47.

The sleep state acquisition unit 41 acquires sleep state information, which is information indicating the sleep state of the user, from at least one of the respiratory information, the body movement information, and the heartbeat information acquired by the sensor unit 11. The sleep state refers to the state of sleep of the user. The sleep state may include, for example, the state of sleep such as sleep duration and sleep stage, and biological information of the user other than the state of sleep, such as fatigue level and stress level. For example, the sleep state information includes at least one of the bedtime, wake-up time, proportion of sleep stages, sleep onset latency (time taken to fall asleep), sleep efficiency, number of nocturnal awakenings, and nocturnal awakening duration of the user.

The bedtime refers to the time the user goes to bed. The wake-up time refers to the time the user wakes up the next morning or the like. The sleep stage is an index indicating the depth of sleep of the user. The sleep stages are classified into, for example, awakening, REM sleep, and non-REM sleep. The non-REM sleep is classified into, for example, four types of sleep stages. Incidentally, the non-REM sleep may be classified into two or three types of sleep stages. Hereinafter, the stage of REM sleep or non-REM sleep may be referred to as "sleep". The time the sleep stage of the user transitions from awakening to sleep between the bedtime and the wake-up time is referred to as the sleep onset time. The time the sleep stage of the user finally becomes an awake stage between the bedtime and the wake-up time is referred to as the awakening time.

The proportion of sleep stages refers to the proportion of the time spent in each sleep stage with respect to the time from the bedtime to the wake-up time. The sleep onset latency (time taken to fall asleep) refers to the length of time from the bedtime to the sleep onset time. The sleep efficiency is an index for evaluating the quality of sleep. The sleep efficiency is, for example, a value obtained by dividing the time, which is obtained by subtracting the nocturnal awakening duration from the time from the sleep onset time to the awakening time, by the time from the bedtime to the sleep onset time. The nocturnal awakening refers to the awakening of the user between the sleep onset time and the awakening time. The term "awakening" refers to the transition of the sleep stage from the stage of REM sleep or non-REM sleep to an awake stage.

For example, the sleep state acquisition unit 41 determines the bedtime and the wake-up time based on the body movement information acquired by the sensor unit 11 and the movement of bedding detected by the sensor unit 11 (for example, an acceleration sensor), and acquires the bedtime and the wake-up time. The sleep state acquisition unit 41 acquires, for example, the sleep stage using the signal indicating body movement associated with respiration, the signal indicating body movement associated with heartbeat, and the signal indicating body movement not associated with respiration and heartbeat that are output from the sensor unit 11.

The sleep state acquisition unit 41 acquires, for example, the proportion of sleep stages from the sleep stage, the bedtime, and the wake-up time. The sleep state acquisition unit 41 acquires, for example, the time from the bedtime to the sleep onset time as the sleep onset latency based on the bedtime and the sleep stage.

The sleep state acquisition unit 41 acquires, for example, the nocturnal awakening duration and the number of nocturnal awakenings from the bedtime, the wake-up time, and the sleep stage. For example, the sleep state acquisition unit 41 acquires the sleep onset time from the bedtime and the sleep stage, and acquires the awakening time from the wake-up time and the sleep stage. The sleep state acquisition unit 41 acquires the nocturnal awakening duration and the number of nocturnal awakenings from the sleep onset time, the awakening time, and the sleep stage.

The sleep state acquisition unit 41 acquires, for example, the sleep efficiency from the bedtime, the wake-up time, and the sleep stage. The sleep state acquisition unit 41 acquires, for example, the sleep efficiency from the bedtime, the wake-up time, the sleep onset time, the awakening time, and the nocturnal awakening duration.

The autonomic nervous system acquisition unit 42 acquires autonomic nervous system information, which is information indicating the state of the autonomic nervous system of the user, from the heartbeat information acquired by the sensor unit 11. The autonomic nervous system refers to nerves that control the activities of organs such as the heart or stomach or involuntary functions such as blood circulation. The term "involuntary" refers to, for example, a situation in which something does not proceed at will or does not occur in accordance with one's own intention. The autonomic nervous system is composed of the sympathetic nervous system and the parasympathetic nervous system. The sympathetic nervous system and the parasympathetic nervous system are regulated in balance with each other.

The sympathetic nervous system is activated when the user is excited, tense, or stressed. When the sympathetic nervous system is activated, responses such as faster heartbeat, shallower and faster respiration, constricted blood vessels, and increased blood pressure appear in the body. The parasympathetic nervous system is activated when the user is relaxed. When the parasympathetic nervous system is activated, responses such as slower heartbeat, deeper and slower respiration, dilated blood vessels, lowered blood pressure, and enhanced immune function appear in the body. Such autonomic nervous system responses are responses that cannot be intentionally controlled by a person's intention, and are used as objective indicators of emotions, feelings, fatigue, stress, and the like.

The autonomic nervous system acquisition unit 42 acquires, for example, the heart rate variability (HRV) of the user by analyzing the heartbeat information acquired by the sensor unit 11. For example, the autonomic nervous system acquisition unit 42 acquires, from the heart rate variability of the user, information indicating periodic components included in the heart rate variability. The autonomic nervous system acquisition unit 42 performs frequency analysis on the periodic components of the heart rate variability to acquire information indicating the power spectrum of each frequency.

The power spectrum of the autonomic nervous system obtained as a result of the frequency analysis is divided into a low frequency (LF) component that is the integral value of the power spectrum in a low-frequency band (as one example, 0.04 Hz to 0.15 Hz), and a high frequency (HF) component that is the integral value of the power spectrum in a high-frequency band (as one example, 0.15 Hz to 0.4 Hz). The LF component reflects sympathetic nervous system activity and parasympathetic nervous system activity, and the HF component reflects parasympathetic nervous system activity. The autonomic nervous system acquisition unit 42 acquires, as the autonomic nervous system information, information indicating a sympathetic nervous system index and information indicating a parasympathetic nervous system index. The sympathetic nervous system index is an index indicating the dominance of the sympathetic nervous system. The sympathetic nervous system index is, as one example, a value obtained by dividing the value of the LF component by the value of the HF component. The parasympathetic nervous system index is an index indicating the dominance of the parasympathetic nervous system. The parasympathetic nervous system index is, as one example, a value obtained by dividing the value of the HF component by the sum of the LF component and the HF component.

The autonomic nervous system acquisition unit 42 acquires the mental state of the user from the autonomic nervous system information. The autonomic nervous system acquisition unit 42 classifies, for example, the mental state of the user as one of a high-performance state, a relaxed state, a stressed state, and a depressed state, based on the autonomic nervous system information.

The high-performance state refers to, for example, a state in which the sympathetic nervous system index is equal to or greater than a predetermined first threshold value and the parasympathetic nervous system index is equal to or greater than a predetermined second threshold value. The relaxed state refers to, for example, a state in which the sympathetic nervous system index is less than the first threshold value and the parasympathetic nervous system index is equal to or greater than the second threshold value. The stressed state refers to, for example, a state in which the sympathetic nervous system index is equal to or greater than the first threshold value and the parasympathetic nervous system index is less than the second threshold value. The depressed state refers to, for example, a state in which the sympathetic nervous system index is less than the first threshold value and the parasympathetic nervous system index is less than the second threshold value.

For example, the lifestyle improvement application 40 generates information indicating the exercise performance of the user from past sleep information stored in the lifestyle improvement server 102, the autonomic nervous system information acquired by the autonomic nervous system acquisition unit 42, and the information indicating body temperature, the information indicating blood pressure, and the information indicating heart rate that are acquired by the sensor unit 11. The past sleep information refers to past sleep state information of the user. The past refers to a time before the time the device control unit 44 controls the operation of a device 104. The past sleep information is, for example, accumulated data of sleep information of the user from several days ago to the previous day.

The exercise performance is an index indicating the physical condition of the user. The lifestyle improvement application 40 generates information indicating thinking ability from the past sleep information, the autonomic nervous system information, and the respiratory information. The lifestyle improvement application 40 generates, for example, information indicating concentration and information indicating sleepiness from the autonomic nervous system information. The lifestyle improvement application 40 generates information indicating response speed from the autonomic nervous system information and the body movement information. The lifestyle improvement application 40 may quantify each of the exercise performance, thinking ability, concentration, and response speed of the user. The lifestyle improvement application 40 generates, for example, information indicating the number of times of turning over during sleep from the past sleep information and the body movement information.

The storage unit 43 stores the sleep state information acquired by the sleep state acquisition unit 41. For example, the storage unit 43 stores the sleep state information in the lifestyle improvement server 102. The storage unit 43 may store, for example, the sleep state information in the memory of the information terminal 101.

The device control unit 44 controls the operation of the device 104 based on at least one of the past sleep information stored in advance in the lifestyle improvement server 102 and the autonomic nervous system information acquired by the autonomic nervous system acquisition unit 42. The device control unit 44 may determine, for example, whether the proportion of REM sleep is equal to or greater than a predetermined value, based on the past sleep information. The device control unit 44 may determine, for example, whether the sleep efficiency is equal to or greater than a predetermined value, based on the past sleep information. The device control unit 44 may determine, for example, whether the average value of the time from the bedtime to the sleep onset time (sleep onset latency) in the past sleep information is equal to or greater than a predetermined time.

The device control unit 44 may determine, for example, whether the number of times of turning over during sleep is equal to or greater than a predetermined value, based on the information indicating the number of times of turning over during sleep. The device control unit 44 may determine, for example, whether the sympathetic nervous system index is equal to or greater than a predetermined value, based on the autonomic nervous system information. The device control unit 44 may determine, for example, whether the number of nocturnal awakenings acquired by the sleep state acquisition unit 41 is equal to or greater than a predetermined value. The device control unit 44 may determine, for example, whether the exercise performance of the user is equal to or greater than a predetermined value, based on the information indicating exercise performance. The device control unit 44 may determine, for example, whether the thinking ability of the user is equal to or greater than a predetermined value, based on the information indicating thinking ability. The device control unit 44 may determine, for example, whether the response speed of the user is equal to or greater than a predetermined value, based on the information indicating response speed. The device control unit 44 may control the operation of the device 104 based on at least one of the determination results described above.

The lifestyle improvement system 1 further includes a device server 105. The device server 105 can communicate with the device control unit 44. The device server 105 receives information for controlling the operation of the device 104 from the device control unit 44. The device server 105 controls the device 104 based on the received information. However, when the device control unit 44 can directly communicate with the device 104, the device server 105 may not be provided. The device server 105 may be able to communicate with the lifestyle improvement server 102. In this case, the device server 105 may communicate with the information terminal 101 via the lifestyle improvement server 102.

The device 104 constitutes the environment surrounding the user. The device 104 includes, for example, at least one of an air conditioner installed in the bedroom of the user, a lighting fixture, a pillow used by the user for sleeping, a heated mattress, a coffee maker, and an automobile.

For example, when the device 104 is an air conditioner, the device control unit 44 may control the operation of the air conditioner to adjust the temperature of a space where the user is present. For example, when it is determined that the proportion of REM sleep is equal to or greater than the predetermined value, the device control unit 44 may control the device 104 to increase the temperature of the space to warm the space.

For example, when the device 104 is a lighting fixture, the device control unit 44 may control the operation of the lighting fixture to adjust at least one of the brightness and color temperature of a space where the user is present. For example, when it is determined that the sleep efficiency is not equal to or greater than the predetermined value, the device control unit 44 may control the device 104 to brighten the space, in which the user is present, in the morning such as the next morning. For example, when the mental state of the user is classified as the relaxed state or the depressed state by the autonomic nervous system acquisition unit 42, the device control unit 44 may execute the same control as described above. In this case, the space can be brightened, which contributes to improving the mental state of the user and the like.

For example, when the device 104 is a pillow, the device control unit 44 may control the operation of the pillow to control the angle of the placement surface of the pillow, on which the head of the user is placed, with respect to a horizontal plane. For example, when it is determined that the average value of the time from the bedtime to the sleep onset time in the past sleep information is equal to or greater than the predetermined time, the device control unit 44 may control the device 104 to reduce the angle of the placement surface with respect to the horizontal plane. For example, when it is determined that it is necessary to secure the airway, based on the past sleep information and the respiratory information, the angle of the head and neck of the user with respect to the horizontal plane can be adjusted, and therefore, the tilt of the pillow can be adjusted such that the head of the user is positioned for side sleeping. Accordingly, snoring of the user can be suppressed.

For example, when the device 104 is a heated mattress, the device control unit 44 may control the operation of the heated mattress to adjust temperature in the bed of the user. For example, when it is determined that the number of times of turning over during sleep is not equal to or greater than the predetermined value and the sleep efficiency is not equal to or greater than the predetermined value, the device control unit 44 may control the device 104 to increase temperature in the bed. For example, when it is determined that the number of nocturnal awakenings is equal to or greater than a predetermined value, the device control unit 44 may control the device 104 to increase temperature in the bed in real time.

For example, when the device 104 is a coffee maker, the device control unit 44 may control the operation of the coffee maker to adjust the strength of the coffee that the user drinks after awakening. For example, when it is determined that the sympathetic nervous system index is not equal to or greater than the predetermined value, the device control unit 44 may control the device 104 to increase the strength of the coffee.

For example, when the device 104 is an automobile, the automobile may execute vehicle control in a plurality of driving modes. The plurality of driving modes include, for example, a steering assist mode in which steering by a driver is assisted (autonomous driving mode) and a normal mode in which steering assistance is not performed. For example, the device control unit 44 may control the automobile to switch the driving mode of the automobile to the steering assist mode or the normal mode or recommend the user to perform switching. For example, when it is determined that the exercise performance is not equal to or greater than the predetermined value, the device control unit 44 may switch the operation of the automobile to the steering assist mode, or recommend the user to perform switching. For example, when it is determined that the thinking ability of the user is not equal to or greater than the predetermined value, the device control unit 44 may execute the same control as described above. For example, when it is determined that the response speed of the user is not equal to or greater than the predetermined value, the device control unit 44 may execute the same control as described above. In other cases, for example, the device control unit 44 may switch the operation of the automobile to the normal mode, or recommend the user to perform switching.

The prediction information generation unit 45 generates the prediction information, which is information indicating the predicted state of the user while awake, from at least one of the past sleep information acquired by the sleep state acquisition unit 41 and the autonomic nervous system information acquired by the autonomic nervous system acquisition unit 42. For example, the prediction information includes mental information that is information indicating the mental state of the user, physical condition information that is information indicating the physical condition of the user, and brain information that is information indicating the state of the brain of the user.

The prediction information generation unit 45 may determine, for example, whether the sleep efficiency is equal to or greater than the predetermined value, based on the past sleep information. The prediction information generation unit 45 may determine, for example, whether the concentration of the user is equal to or greater than the predetermined value, based on the information indicating concentration. The prediction information generation unit 45 may generate the prediction information from the determination result of the sleep efficiency and the determination result of the concentration described above.

The prediction information generation unit 45 generates, for example, the mental information from the autonomic nervous system information. The mental information includes, for example, information regarding the mood swings of the user. As one example, the mental information includes information indicating whether the mood of the user tends to become depressed. For example, when the mental state of the user is classified as the stressed state or the depressed state by the autonomic nervous system acquisition unit 42, the prediction information generation unit 45 may generate, as the mental information, the information indicating that the mood of the user tends to become depressed.

The prediction information generation unit 45 generates, for example, the physical condition information from the past sleep information. The physical condition information includes, for example, information indicating whether the physical condition of the user is good while awake. For example, when it is determined that the sleep efficiency is not equal to or greater than the predetermined value, the prediction information generation unit 45 may generate, as the physical condition information, information indicating that the physical condition of the user tends to decline. For example, the physical condition information includes skin information indicating the state of the skin of the user. The skin information includes, for example, information indicating that the skin quality of the user is predicted to be poor. The term "skin quality being poor" refers to, for example, a state in which the moisture content of the skin decreases below a certain value.

The prediction information generation unit 45 generates, for example, the brain information from the past sleep information and the autonomic nervous system information. The brain information is information indicating whether the brain of the user functions well. The brain information includes, for example, information indicating whether the concentration, memory, and thinking ability of the user are good. For example, when the mental state of the user is classified as the high-performance state by the autonomic nervous system acquisition unit 42, the prediction information generation unit 45 may generate, as the brain information, information indicating that the concentration of the user is predicted to be high. For example, when it is determined that the sleep efficiency is not equal to or greater than the predetermined value, the prediction information generation unit 45 may generate, as the brain information, information indicating that the thinking ability of the user is predicted to be low. For example, when the mental state of the user is classified as the stressed state, the relaxed state, or the depressed state by the autonomic nervous system acquisition unit 42, or even when it is determined that the concentration of the user is not equal to or greater than the predetermined value, the prediction information generation unit 45 may execute the same processing described above.

The improvement information generation unit 46 generates the improvement information, which is information for improving the lifestyle of the user, from the prediction information generated by the prediction information generation unit 45. For example, the improvement information includes at least one of information indicating the type of clothing recommended for the user, information indicating the types of soap, cosmetics, and food, and information indicating a recommended behavior. The information indicating the type of clothing includes, for example, at least one of information on the color of the clothing, information on the type of fabric of the clothing, and the number of pieces of clothing to be worn.

For example, the improvement information generation unit 46 generates, as the improvement information, the information indicating the type of cosmetics from the skin information generated by the prediction information generation unit 45. For example, when the prediction information generation unit 45 generates the information indicating that the skin quality is predicted to be poor, the improvement information generation unit 46 generates information indicating the type of cosmetics that enhance the moisturizing ability of the skin.

For example, the improvement information generation unit 46 generates, as the improvement information, information indicating the type (for example, color) of apparel recommended for the user from the information indicating that the mood of the user is predicted to tend to become depressed. As one example, when the mental state of the user is classified as the depressed state by the autonomic nervous system acquisition unit 42, the improvement information generation unit 46 may generate improvement information indicating that warm-colored (for example, red) apparel is recommended. For example, the improvement information generation unit 46 generates, as the improvement information, at least one of information indicating the types of soap, cosmetics, and food recommended for the user and information indicating that the user has to avoid sunlight during the day, from the information indicating that the skin quality of the user is predicted to be poor.

For example, the improvement information generation unit 46 generates, as the improvement information, information indicating that the user is recommended not to make an important decision, from the information indicating that the thinking ability of the user is predicted to be low. For example, the improvement information generation unit 46 generates, as the improvement information, information indicating whether the steering assist mode or the normal mode is recommended as the driving mode of an automobile, from the information indicating exercise performance, the information indicating thinking ability, and the information indicating response speed.

The arrhythmia detection unit 47 detects the presence or absence of arrhythmia in the user from the heartbeat information acquired by the sensor unit 11. Arrhythmia is, for example, a respiratory arrhythmia. In respiratory arrhythmia, the interval between heartbeats shortens when breathing in and lengthens when breathing out. In this case, when breathing in, it is desirable to increase oxygen concentration and blood flow rate to take in a large amount of oxygen, and when breathing out, it is desirable to decrease oxygen concentration and blood flow rate to promote recovery from cardiac fatigue.

The term "arrhythmia" refers to any of a slow pulse, a fast pulse, and an irregular pulse. The term "arrhythmia" refers to a state in which the speed or rhythm of the heartbeat (the contraction and dilation of the heart) is disturbed. The term "pulse" refers to the rhythm at which the heart pumps blood throughout the body, namely, the number of times the heart beats in a certain period of time. When arrhythmia occurs, symptoms such as palpitations, chest tightness, shortness of breath, fatigue, dizziness, fainting, and skipped pulses occur. Arrhythmia is often not dangerous. However, since arrhythmia may also be caused by heart disease, it is desirable to be able to detect arrhythmia appropriately.

Arrhythmia includes bradyarrhythmia, tachyarrhythmia, and premature contraction. In bradyarrhythmia, the heartbeat is slow (for example, 50 BPM or less), and a person is likely to feel shortness of breath or fatigue. When the heartbeat becomes even slower, there is a possibility that dizziness or fainting occurs. Arrhythmia includes those caused by sick sinus syndrome and those caused by atrioventricular block. In arrhythmia caused by sick sinus syndrome, an abnormality occurs in the sinus node, and therefore, the frequency of generation of electrical signals decreases and the heartbeat is slow. In arrhythmias caused by atrioventricular block, electrical signals have difficulty transmitting through or become interrupted in the pathways connecting the atria and ventricles.

In tachyarrhythmia, the heartbeat is fast (for example, 100 BPM or more), and a person is likely to feel palpitations or chest tightness. Arrhythmia includes those caused by atrial flutter, those caused by atrial fibrillation, those caused by ventricular tachycardia, and those caused by ventricular fibrillation. In arrhythmia caused by atrial flutter, the atria beat rapidly, and the heart rate becomes approximately 300 BPM. In arrhythmia caused by atrial fibrillation, the atria beat rapidly, and the heart rate becomes approximately 500 BPM. When arrhythmia caused by atrial fibrillation continues for a long period of time, there is a possibility that blood clots form in the atria and a cerebral infarction occurs. In arrhythmia caused by ventricular tachycardia, abnormal electrical impulses occur in the ventricles, and the heart rate becomes approximately 200 BPM. In arrhythmia caused by ventricular fibrillation, abnormal electrical impulses occur in the ventricles, and the heart rate becomes approximately 400 BPM.

For example, the arrhythmia detection unit 47 detects various arrhythmias described above. The arrhythmia detection unit 47 classifies, for example, the waveform of the heartbeat into a normal waveform and an abnormal waveform. The arrhythmia detection unit 47 detects the abnormal waveform obtained as a result of the classification as an arrhythmia. The arrhythmia detection unit 47 may learn the normal waveform and the abnormal waveform. In this case, the arrhythmia detection unit 47 can efficiently determine whether the waveform of the heartbeat is a normal waveform or an abnormal waveform.

The arrhythmia detection unit 47 detects, for example, the timing of the occurrence of arrhythmia from the heartbeat information. The arrhythmia detection unit 47 may calculate the frequency of arrhythmia from the heartbeat information. The arrhythmia detection unit 47 may calculate the duration of arrhythmia from the heartbeat information. The arrhythmia detection unit 47 may detect the presence or absence of premature contraction from the heartbeat information. The term "premature contraction" refers to a case in which an irregular heartbeat occurs between normal heartbeats. The term "premature contraction" may also refer to a case in which a heartbeat occurs earlier than expected due to a timing shift occurring between heartbeats occurring at a constant rhythm. For example, when the arrhythmia detection unit 47 detects a heart rate equal to or greater than X times (X is a positive real number) the immediately preceding heart rate, or when the arrhythmia detection unit 47 detects a heart rate less than or equal to 1/X times the immediately preceding heart rate, the arrhythmia detection unit 47 detects the presence of a premature contraction. As one example, the value of X is 2.

The prediction information generation unit 45 may include information on arrhythmia detected by the arrhythmia detection unit 47 in the prediction information. For example, the prediction information generation unit 45 may predict a disease that may occur in the user in the future, based on the information on arrhythmia detected by the arrhythmia detection unit 47, and include the predicted disease in the prediction information. The improvement information generation unit 46 may generate, as the improvement information, information for eliminating arrhythmia from the information on arrhythmia detected by the arrhythmia detection unit 47. For example, the improvement information generation unit 46 includes, in the improvement information, information for recommending any of getting more sleep, reducing alcohol intake, or visiting a hospital (as one example, a cardiology department). The improvement information generation unit 46 may include, in the improvement information, advice indicating that dangerous work and driving an automobile should be avoided. The improvement information generation unit 46 may include, in the improvement information, advice indicating that at least one of a respiration technique and a relaxation technique should be incorporated.

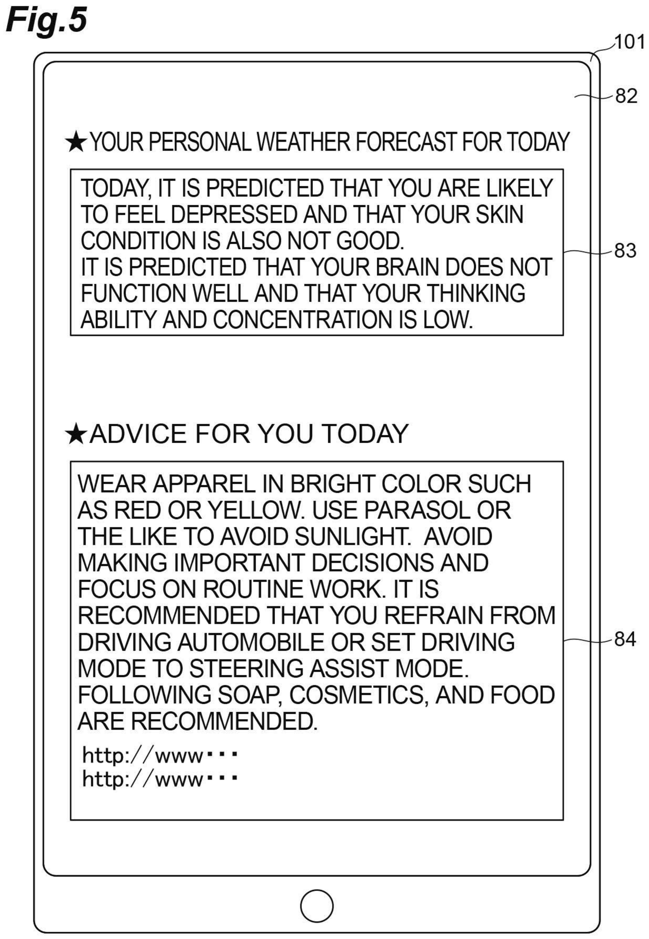

For example, the lifestyle improvement application 40 displays the prediction information generated by the prediction information generation unit 45 and the improvement information generated by the improvement information generation unit 46 on the information terminal 101. FIG. 5 is a view showing an example of an output screen 82 for the prediction information and the improvement information displayed on the information terminal 101. The lifestyle improvement application 40 displays, for example, the prediction information and the improvement information on the information terminal 101 as the output screen 82. The output screen 82 is, for example, a screen for providing the prediction information and the improvement information to the user. The output screen 82 includes a prediction information display portion 83 that is a portion for displaying the prediction information, and an improvement information display portion 84 that is a portion for displaying the improvement information.

The prediction information display portion 83 displays, for example, at least one of the information indicating that the mood of the user is predicted to tend to become depressed, the information indicating that the skin quality is predicted to be poor, and the information indicating that the concentration and the thinking ability are predicted to be low. The prediction information display portion 83 may display, for example, at least one of information indicating that the mood of the user is predicted to tend to be elevated, information indicating that the skin quality is predicted to be good, and information indicating that the concentration and the thinking ability are predicted to be high.

The improvement information display portion 84 displays, for example, at least one of the information indicating the color of apparel recommended for the user, the information indicating that the user has to avoid sunlight during the day, the information indicating that the user is recommended not to make an important decision, and the information indicating that the steering assist mode is recommended as the driving mode of an automobile. The improvement information display portion 84 displays, for example, the information indicating soap, cosmetics, and food recommended for the user. The improvement information display portion 84 displays, for example, the URL of the website 103 on which information regarding products recommended for the user is posted.

For example, the improvement information generation unit 46 generates the improvement information from the prediction information and the subjective information described above. The improvement information generation unit 46 may acquire objective information from the past sleep information and the autonomic nervous system information. The objective information refers to objective information of the user obtained from objective data obtained by measurement, such as the sleep state information or the autonomic nervous system information. The objective information includes, for example, an objective evaluation of sleep. The improvement information generation unit 46 acquires a subjective evaluation of sleep included in the subjective information. The improvement information generation unit 46 generates, as the improvement information, feedback information from the subjective evaluation of sleep and the objective evaluation of sleep. The feedback information includes, for example, information indicating a difference between the subjective evaluation of sleep and the objective evaluation of sleep.

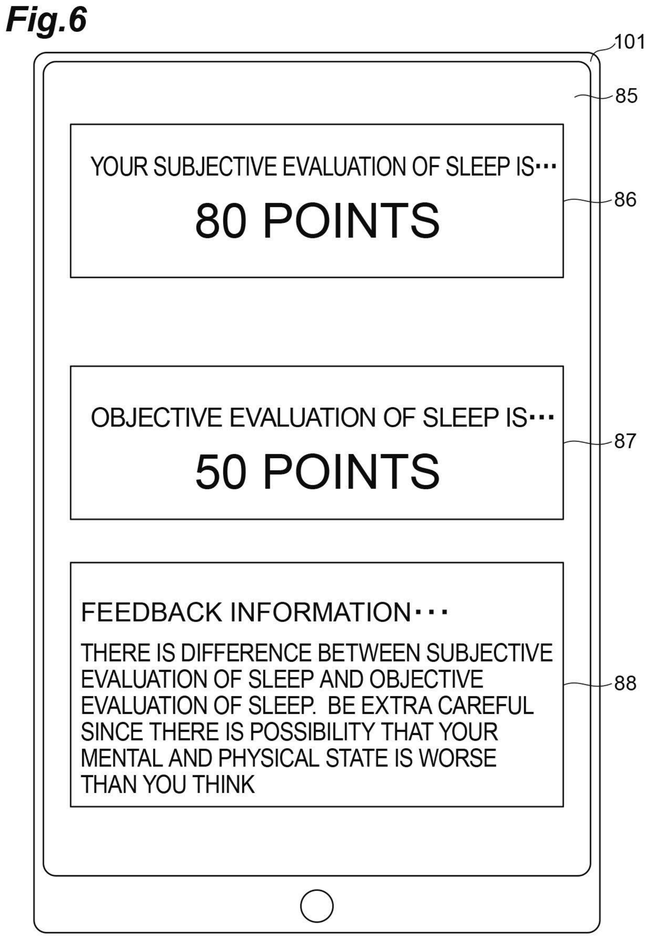

For example, the lifestyle improvement application 40 displays the feedback information generated by the improvement information generation unit 46 on the information terminal 101. FIG. 6 is a view showing an example of an output screen 85 for the feedback information displayed on the information terminal 101. The lifestyle improvement application 40 displays, for example, the feedback information on the information terminal 101 as the output screen 85. The output screen 85 is, for example, a screen for providing the feedback information to the user. The output screen 85 includes a subjective information display portion 86 that displays the subjective evaluation of sleep; an objective information display portion 87 that displays the objective evaluation of sleep; and a feedback display portion 88 that displays the feedback information.

The subjective information display portion 86 displays the subjective information. The subjective information display portion 86 displays, for example, the subjective evaluation of sleep that is input by the user to the sleep evaluation input portion 81 (see FIG. 2). The objective information display portion 87 displays the objective information. The objective information display portion 87 displays, for example, the objective evaluation of sleep of the user obtained from the sleep state information acquired by the sleep state acquisition unit 41 and the autonomic nervous system information acquired by the autonomic nervous system acquisition unit 42. The objective information display portion 87 displays, for example, the objective evaluation of sleep as a score with a maximum score of 100 points. The feedback display portion 88 displays, for example, the information indicating the difference between the subjective evaluation of sleep and the objective evaluation of sleep.

The lifestyle improvement application 40 performs total feedback. The total feedback includes feedback for improving the accuracy of control of the device 104. The total feedback is performed to improve the accuracy of the generation of the prediction information and the improvement information. For example, when the subjective evaluation of sleep is higher than the objective evaluation of sleep by a predetermined value or more, the device control unit 44 may prioritize control of the device 104 based on subjective assessment over control of the device 104 based on objective assessment. In this case, control of the device 104 in accordance with subjective assessment of the user can be realized.

Communication between the sensor unit 11 and the lifestyle improvement application 40, communication between the lifestyle improvement application 40 and the lifestyle improvement server 102, communication between the lifestyle improvement server 102 and the information terminal 101, communication between the lifestyle improvement server 102 and the device server 105, communication between the device control unit 44 and the device server 105, and communication between the device server 105 and the device 104 may be realized, for example, by a wireless communication interface such as a wireless Local Area Network (LAN) or Bluetooth (registered trademark). In addition, these communications may be realized by wire.

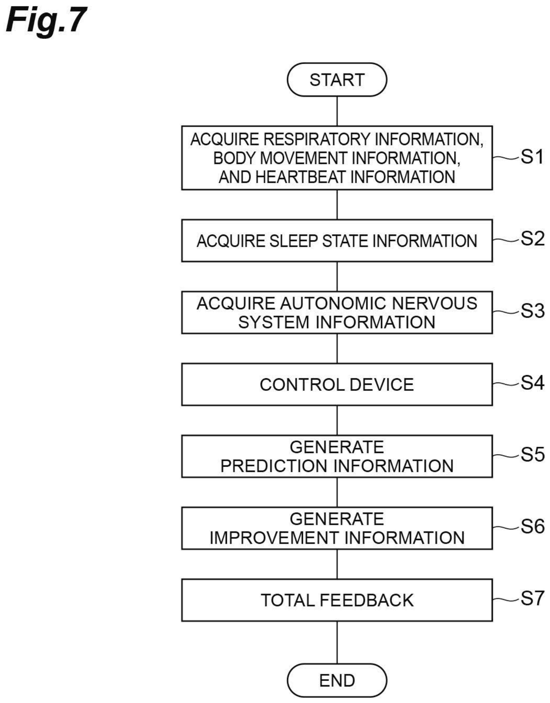

One example of the operation of the lifestyle improvement system 1 will be described. FIG. 7 is a flowchart showing one example of the operation of the lifestyle improvement system 1. Before the lifestyle improvement system 1 is operated, first, the sensor sheet 12 is attached to the mattress 10a, and the cushion sensor 13 is attached to the cushion 10b. Next, the body of the user is placed on the mattress 10a or the cushion 10b. For example, during sleep, the body of the user is placed on the mattress 10a so as to come into contact with the front fabric 31 of the cover fabric 3. For example, during the day, the body of the user is placed on the seating portion 15 of the cushion 10b.

The sensor unit 11 acquires the respiratory information, the body movement information, and the heartbeat information from body movement associated with the respiration of the user, body movement associated with heartbeat, and body movement not associated with respiration and heartbeat (step S1). When vital data of the user is acquired by the sensor sheet 12, in step S1, the sensor sheet 12 detects the body movement associated with the respiration of the user, the body movement associated with heartbeat, and the body movement not associated with respiration and heartbeat. The sensor sheet 12 outputs a signal indicating the body movement associated with respiration, a signal indicating the body movement associated with heartbeat, and a signal indicating the body movement not associated with respiration and heartbeat to the outside of the sensor sheet 12. When vital data of the user is acquired by the cushion sensor 13, in step S1, the cushion sensor 13 detects body movement associated with the heartbeat of the user. The cushion sensor 13 outputs a signal indicating the body movement associated with heartbeat to the outside of the cushion sensor 13. In such a manner, the sensor unit 11 outputs the signal indicating the body movement associated with respiration, the signal indicating the body movement associated with heartbeat, and the signal indicating the body movement not associated with respiration and heartbeat to the outside of the sensor unit 11.

The sleep state acquisition unit 41 acquires, for example, the sleep state information from the signals output from the sensor unit 11. The sleep state acquisition unit 41 acquires the sleep state information of the user from at least one of the respiratory information, the body movement information, and the heartbeat information acquired by the sensor unit 11 (step S2). In step S2, the sleep state acquisition unit 41 acquires, for example, the bedtime, wake-up time, proportion of sleep stages, sleep onset latency, sleep efficiency, number of nocturnal awakenings, and nocturnal awakening duration of the user from the respiratory information, the body movement information, and the heartbeat information.

The autonomic nervous system acquisition unit 42 acquires, for example, the autonomic nervous system information from the heartbeat information acquired by the sensor unit 11 (step S3). In step S3, for example, the autonomic nervous system acquisition unit 42 acquires the heart rate variability of the user by analyzing the heartbeat information acquired by the sensor unit 11. In step S3, the autonomic nervous system acquisition unit 42 acquires the autonomic nervous system information from the heart rate variability of the user. The autonomic nervous system information includes, for example, information indicating the frequency of the R wave of the heartbeat. In step S3, the autonomic nervous system acquisition unit 42 acquires the mental state of the user from the autonomic nervous system information.

For example, the device control unit 44 controls the operation of the device 104 based on at least one of the past sleep information and the autonomic nervous system information (step S4). In step S4, for example, the device control unit 44 controls the operation of a coffee maker. The device control unit 44 may control, for example, the operation of the coffee maker to adjust the strength of the coffee that the user drinks after awakening, in accordance with the past sleep information and the mental state of the user.

The prediction information generation unit 45 generates the prediction information from at least one of the past sleep information stored in advance in the lifestyle improvement server 102 and the autonomic nervous system information acquired by the autonomic nervous system acquisition unit 42 (step S5). In step S5, for example, the prediction information generation unit 45 generates, as the prediction information, at least one of the mental information, the physical condition information, and the brain information from the past sleep information and the autonomic nervous system information. The prediction information generation unit 45 generates, for example, the skin information as the physical condition information. For example, the prediction information generation unit 45 generates, as the prediction information, the information indicating that the mood of the user is predicted to tend to become depressed. For example, the prediction information generation unit 45 generates, as the prediction information, the information indicating whether the physical condition of the user is good while awake. For example, the prediction information generation unit 45 generates, as the prediction information, the information indicating that the concentration and the thinking ability of the user are predicted to be high.

Next, the improvement information generation unit 46 generates the improvement information from the prediction information generated by the prediction information generation unit 45 (step S6). For example, the improvement information generation unit 46 generates, as the improvement information, the information indicating the type of clothing recommended for the user. For example, the improvement information generation unit 46 generates, as the improvement information, the information indicating the type of soap, cosmetics, and food. For example, the improvement information generation unit 46 generates, as the improvement information, the information indicating a recommended behavior.

In step S6, the improvement information generation unit 46 generates the improvement information from the prediction information and the subjective information. The improvement information generation unit 46 acquires, for example, the objective evaluation of sleep included in the objective information obtained from the sleep state information and the autonomic nervous system information. The improvement information generation unit 46 acquires, for example, the subjective evaluation of sleep included in the subjective information input by the user. The improvement information generation unit 46 generates, for example, the feedback information from the subjective evaluation of sleep and the objective evaluation of sleep. The improvement information generation unit 46 generates, for example, the information indicating the difference between the subjective evaluation of sleep and the objective evaluation of sleep.

The lifestyle improvement application 40 displays the prediction information generated by the prediction information generation unit 45 and the improvement information generated by the improvement information generation unit 46. The lifestyle improvement application 40 displays, for example, the prediction information and the improvement information on the information terminal 101 as the output screen 82 (see FIG. 5). Next, the lifestyle improvement application 40 displays the feedback information generated by the improvement information generation unit 46. The lifestyle improvement application 40 displays, for example, the feedback information on the information terminal 101 as the output screen 85 (see FIG. 6).

Subsequently, the lifestyle improvement application 40 performs total feedback (step S7). In step S7, for example, when the subjective evaluation of sleep is higher than the objective evaluation of sleep by the predetermined value or more, the device control unit 44 of the lifestyle improvement application 40 prioritizes control of the device 104 on subjective assessment over control of the device 104 based on objective assessment.

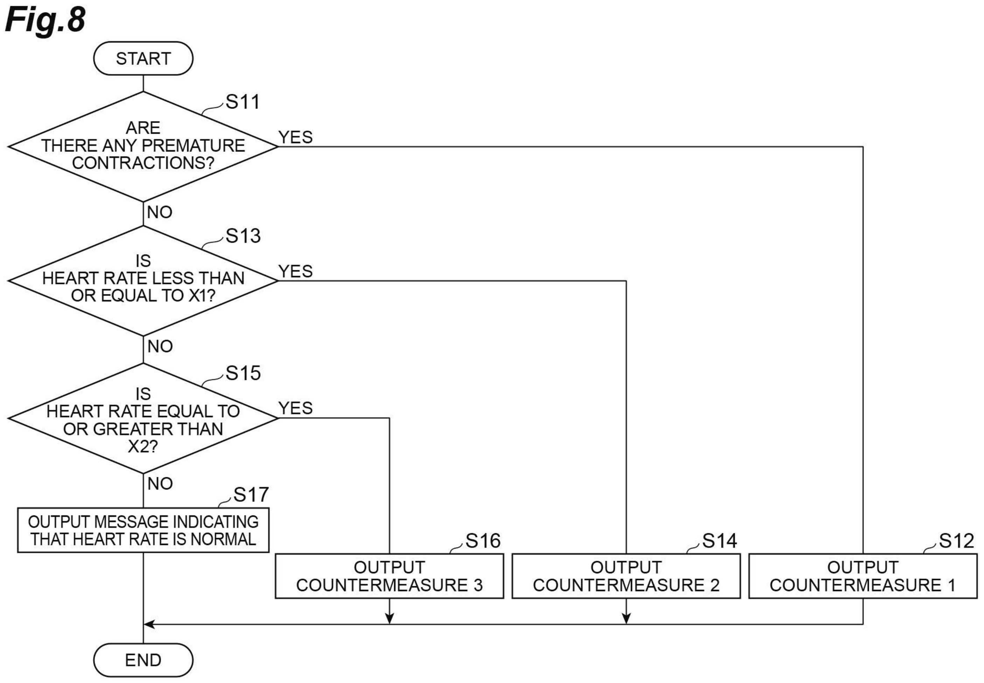

An example of a step of detecting arrhythmia by the arrhythmia detection unit 47 will be described with reference to FIG. 8. The step of detecting arrhythmia is executed, for example, before the prediction information described above is generated (step S5). However, the timing for executing the step of detecting arrhythmia is not particularly limited.

The arrhythmia detection unit 47 detects the presence or absence of arrhythmia from the heartbeat information acquired by the sensor unit 11. The arrhythmia detection unit 47 detects the presence or absence of premature contraction from the heartbeat information (step S11). When the arrhythmia detection unit 47 detects the presence of a premature contraction, the arrhythmia detection unit 47 outputs, for example, Countermeasure 1 (step S12). Countermeasure 1 is advice to encourage the user to improve lifestyle habits, such as getting more sleep or reducing alcohol intake. When the arrhythmia detection unit 47 detects the presence of premature contractions a plurality of times (for example, when premature contractions are detected in 10 or more out of 100 heartbeats), Countermeasure 1 may be advice to encourage the user to visit a cardiology department. For example, Countermeasure 1 output by the arrhythmia detection unit 47 is included as the improvement information by the improvement information generation unit 46, and is displayed as the improvement information on the information terminal 101 (see FIG. 5). Then, a series of the steps are completed.

When the arrhythmia detection unit 47 does not detect premature contraction (NO in step S11), the arrhythmia detection unit 47 determines whether the heart rate is less than or equal to X1 (X1 is a positive real number) (step S13). As one example, the value of X1 is 50 (BMP). When it is determined that the heart rate is less than or equal to X1, the arrhythmia detection unit 47 determines the presence of a bradyarrhythmia. At this time, the arrhythmia detection unit 47 outputs Countermeasure 2 (step S14). Countermeasure 2 is, for example, advice to avoid dangerous work and driving due to the possibility of dizziness and fainting. Countermeasure 2 may be advice to encourage the user to visit a cardiology department. For example, Countermeasure 2 output by the arrhythmia detection unit 47 is included as the improvement information by the improvement information generation unit 46, and is displayed as the improvement information on the information terminal 101. Then, a series of the steps are completed.

When it is determined that the heart rate is not less than or equal to X1 (NO in step S13), the arrhythmia detection unit 47 determines whether the heart rate is X2 (X2 is a positive real number) greater than X1 (step S15). As one example, the value of X2 is 100 (BMP). When it is determined that the heart rate is equal to or greater than X2, the arrhythmia detection unit 47 determines the presence of a tachyarrhythmia. At this time, the arrhythmia detection unit 47 outputs Countermeasure 3 (step S16). Countermeasure 3 is, for example, advice indicating that a respiration technique or a relaxation technique such as meditation should be incorporated. When the arrhythmia detection unit 47 determines that the heart rate of the user while at rest is equal to or greater than X2, Countermeasure 3 may be advice to encourage the user to visit a cardiology department. For example, Countermeasure 3 output by the arrhythmia detection unit 47 is included as the improvement information by the improvement information generation unit 46, and is displayed as the improvement information on the information terminal 101. Then, a series of the steps are completed.

When it is determined that the heart rate is not equal to or greater than X2 (NO in step S15), the arrhythmia detection unit 47 determines that the heart rate is normal and there is no arrhythmia. The arrhythmia detection unit 47 outputs a message indicating that the heart rate is normal and there is no arrhythmia (step S17). For example, information indicating that the heart rate is normal and there is no arrhythmia is included as the improvement information by the improvement information generation unit 46, and is displayed as the improvement information on the information terminal 101. Then, a series of the steps are completed.

One example of the steps of the operation of the lifestyle improvement system 1 has been described above. However, the contents and order of the steps of the operation of the lifestyle improvement system 1 are not limited to the above-described example, and can be changed as appropriate. The step of detecting arrhythmia by the arrhythmia detection unit 47 is not limited to the above-described example. For example, the arrhythmia detection unit 47 may determine the respiratory rate in addition to determining the heart rate. Among arrhythmias, respiratory sinus arrhythmia is characterized by a rule in which the pulse becomes faster when breathing in (inhaling) and the pulse becomes slower when breathing out (exhaling). For example, when this rule is not met, the arrhythmia detection unit 47 can determine that there is a risk. For example, in the case of sleep apnea, bradycardia occurs while respiration stops, and tachycardia occurs when respiration resumes. When the arrhythmia detection unit 47 determines the respiratory rate in addition to determining the heart rate, the arrhythmia detection unit 47 can also detect each abnormality described above.

Actions and effects of the lifestyle improvement system 1 will be described. The lifestyle improvement system 1 generates the prediction information of the user from at least one of the past sleep information and the autonomic nervous system information. The lifestyle improvement system 1 can provide, for example, the user with information indicating the mental and physical state of the user as the prediction information. The lifestyle improvement system 1 generates the improvement information from the prediction information. For example, when the mental and physical state of the user is predicted to tend to become poor, the lifestyle improvement system 1 can provide the user with the information indicating that the user is recommended not to make an important decision as the improvement information. The lifestyle improvement system 1 not only can control the device 104 while the user sleeps, but can also predict the state of the user while awake based on the sleep of the user and provide information for improving the lifestyle of the user based on the sleep state.

In the lifestyle improvement system 1, the prediction information and the improvement information can be generated from the past sleep state of the user. The prediction information indicating the predicted state of the user while awake and the improvement information for improving the lifestyle can be generated by taking into account not only the sleep state of that day but also the past sleep state of the user. Compared to when the prediction information and the improvement information are generated only from the sleep state of the user from the time of going to bed to waking up, the prediction information and the improvement information can be generated based on longer-term records. As a result, the prediction information and the improvement information can be generated more accurately.

As described above, the sensor unit 11 may include the cushion sensor 13 attached to the cushion 10b on which the user is seated. In this case, for example, vital data of the user can be continuously acquired not only during sleep but also during the day. Since the prediction information and the improvement information can be generated by taking into account the vital data of the user that is continuously measured during the day, the prediction information and the improvement information can be generated more accurately using the state of the user both when asleep and when awake.

The prediction information may include the mental information that is information indicating the mental state of the user, the physical condition information that is information indicating the physical condition of the user, and the brain information that is information indicating the state of the brain of the user. In this case, the mental, physical condition, and brain states of the user while awake can be predicted. The physical condition information may include the skin information indicating the state of the skin of the user. In this case, the information indicating the state of the skin of the user can be provided as the prediction information.

The improvement information generation unit 46 generates the improvement information from the subjective information, which is information indicating a subjective evaluation of the user, and the prediction information. Accordingly, the improvement information can be generated by taking into account the subjective evaluation of the user, and therefore, the improvement information can be generated more accurately.

The improvement information may include information indicating the type of clothing, food, and cosmetics recommended for the user. In this case, the information indicating the type of clothing, food, and cosmetics recommended for the user can be provided as the improvement information.

The device 104 may include a pillow used by the user for sleeping, and the device control unit 44 may control the angle of the placement surface of the pillow, on which the head of the user is placed, with respect to a horizontal plane. In this case, since the angle of the placement surface of the pillow can be adjusted while the user sleeps, the sleep state of the user can be further improved.

The lifestyle improvement system 1 includes the sensor unit 11 that acquires the heartbeat information that is information indicating the heartbeat of the user; the autonomic nervous system acquisition unit 42 that acquires the autonomic nervous system information that is information indicating the state of the autonomic nervous system of the user, based on the heartbeat information; the prediction information generation unit 45 that generates the prediction information, which is information indicating the predicted state of the user while awake, from the autonomic nervous system information; and the improvement information generation unit 46 that generates the improvement information, which is information for improving the lifestyle of the user, from the prediction information.

As described above, the lifestyle improvement system 1 generates the prediction information of the user from the autonomic nervous system information, and generates the improvement information from the prediction information. Therefore, the state of the user while awake can be predicted, and the information for improving the lifestyle of the user can be provided.

The lifestyle improvement system 1 may include the arrhythmia detection unit 47 that detects the presence or absence of arrhythmia in the user from the heartbeat information acquired by the sensor unit 11. In this case, information on the presence or absence of arrhythmia while asleep or while awake can be acquired by detecting the presence or absence of arrhythmia from the heartbeat information acquired by the sensor unit 11. The user can identify the presence or absence of his or her own arrhythmia.

An example of the lifestyle improvement system according to the present disclosure has been described above. However, the present disclosure is not limited to the above-described example, and various modifications can be made without departing from the concept described in the claims.

As described above, the sensor unit 11 may acquire information indicating the body temperature of the user. As one example, the lifestyle improvement system may calculate body temperature by correcting the temperature measured by the sensor unit 11 with a predetermined coefficient (or equation). The lifestyle improvement system may monitor variations in the body temperature of the user acquired by the sensor unit 11. The lifestyle improvement system may display variations in the body temperature of the user, which is acquired by the sensor unit 11, on the information terminal 101. For example, the arrhythmia detection unit 47 described above may detect atrial fibrillation, and the sensor unit 11 may continuously measure the body temperature of the user while asleep. In this case, for example, when the user is female, a certain indication corresponding to a female-specific physical change (for example, a sign of menstruation (ovulation) or pregnancy) can be shown. The user can identify the body temperature at which the user is likely to become pregnant.

When a female tries to become pregnant, basal body temperature may be measured to predict an ovulation period. In the lifestyle improvement system, in such a case, a change in body temperature can be identified from the measurement result of the sensor unit 11. Furthermore, the lifestyle improvement system may detect a change in body temperature caused by an infectious disease using the sensor unit 11. In this case, the user can know a clue to his or her own physical problems. The user may be female or male, and in both cases, the actions and effects described above are obtained.

The lifestyle improvement system may quantify and calculate immune strength from body temperature. It is generally known that immune strength decreases when body temperature decreases. Therefore, the lifestyle improvement system calculates immune strength from body temperature, thereby enabling the user to identify his or her own immune strength and understand his or her own health state. When basal body temperature rises, blood flow improves, shoulder stiffness is alleviated, basal metabolism increases, and the user becomes more likely to lose weight. Furthermore, when basal body temperature rises, swelling is reduced, and menstrual pain and menstrual irregularity are alleviated. Therefore, the lifestyle improvement system calculates basal body temperature from the measurement result of the sensor unit 11, thereby enabling the user to identify tendencies in his or her own blood flow, shoulder stiffness, basal metabolism, whether the user becomes more likely to lose weight, swelling, menstrual pain, and menstrual irregularity.

The sensor unit 11 may include a blood glucose sensor for acquiring blood glucose information that is information indicating the blood glucose level of the user. The blood glucose sensor is provided, for example, on the handrail of a bed on which the mattress 10a on which the user sleeps is disposed. In this case, the prediction information generation unit 45 may generate, as the physical condition information, information indicating the likelihood of a hangover from at least one of the information indicating blood glucose level, the information indicating blood pressure, and the heartbeat information acquired by the sensor unit 11 and the number of nocturnal awakenings acquired by the sleep state acquisition unit 41.

The prediction information generation unit 45 may determine, for example, whether the blood glucose level of the user is equal to or greater than a predetermined value, based on the information indicating blood glucose level. The prediction information generation unit 45 may determine, for example, whether the blood pressure of the user is equal to or greater than a predetermined value, based on the information indicating blood pressure. The prediction information generation unit 45 may determine, for example, whether the heart rate of the user is equal to or greater than a predetermined value, based on the heartbeat information. The prediction information generation unit 45 may determine, for example, whether the number of nocturnal awakenings is equal to or greater than the predetermined value.

For example, when it is determined that the blood glucose level of the user is equal to or greater than the predetermined value, the prediction information generation unit 45 may generate, as the prediction information, information indicating a high likelihood of a hangover. For example, when it is determined that the blood pressure of the user is equal to or greater than the predetermined value, or when it is determined that the heart rate of the user is equal to or greater than the predetermined value, the prediction information generation unit 45 may execute the same processing described above. For example, when it is determined that the number of nocturnal awakenings is equal to or greater than the predetermined value, the prediction information generation unit 45 may execute the same processing described above.

In the above description, an example in which the lifestyle improvement system 1 provides the improvement information to the user has been described; however, the lifestyle improvement system 1 may provide the improvement information to a person other the user. The "person other than the user" may be, for example, a family member of the user. In this case, when the prediction information generation unit 45 generates, as the prediction information, information indicating that the physical condition of the user is predicted to be poor, the lifestyle improvement application 40 may display the prediction information on an information terminal owned by the family member of the user. In this case, since the prediction information of the user can be provided to the family member of the user, the prediction information of the user can be shared with the family member of the user. For example, when the user is an elderly person living alone, the a family member of the user can sense any abnormalities in the user.

The "person other than the user" may be, for example, an industrial physician of the company where the user works. In this case, the prediction information generation unit 45 may determine whether the user is predicted to have an illness, based on the past sleep information and the autonomic nervous system information. When it is determined that the user is predicted to have an illness, the prediction information generation unit 45 may provide, as the prediction information, information indicating that the user is predicted to have an illness to the industrial physician.

The "person other than the user" may be, for example, a real estate company that manages the property, or an insurance company that handles the insurance. In this case, when it is determined that the user is predicted not to have an illness, the prediction information generation unit 45 may provide, as the prediction information, information indicating that the user is predicted not to have an illness to the real estate company or the insurance company. When the prediction information generation unit 45 provides the prediction information to the real estate company, the real estate company can more accurately determine whether a property should be leased to the user. When the prediction information generation unit 45 provides the prediction information to the insurance company, the insurance company can more accurately determine whether an insurance contract should be concluded with the user.

The "person other than the user" may be, for example, an employee of the human resources department of the company where the user works. In this case, the prediction information generation unit 45 may determine, for example, a tendency indicating whether the mental and physical state of the user (each employee of the company) is good, based on the past sleep information and the autonomic nervous system information. For example, when it is determined that the mental and physical state of each employee tends to become poor, the prediction information generation unit 45 may generate, as the prediction information, information indicating that the mental and physical state of each employee tends to become poor. In this case, for example, the improvement information generation unit 46 may generate, as the improvement information, information indicating a recommendation to reduce overtime work.

In the above description, an air conditioner, a lighting fixture, a pillow, a heated mattress, a coffee maker, and an automobile have described as examples of the device 104. However, the device 104 may be, for example, an aroma diffuser that emits a fragrance into the space where the user is present, or a music player. In such a manner, the type of the device 104 is not particularly limited.

The prediction information and the improvement information are not limited to those described above. For example, when it is determined that the thinking ability of the user is not equal to or greater than the predetermined value, the prediction information generation unit 45 may generate, as the brain information, information indicating that the user is predicted to be unsuited to risky behavior (for example, investment). For example, when the mental state of the user is classified as the stressed state or the depressed state by the autonomic nervous system acquisition unit 42, the prediction information generation unit 45 may execute the same processing as described above. For example, the improvement information generation unit 46 may generate, as the improvement information, information indicating a recommendation not to engage in risky behavior.

For example, when it is determined that the thinking ability and the concentration of the user are equal to or greater than the predetermined values, the prediction information generation unit 45 may generate, as the prediction information, information indicating that the user is predicted to be suited to learning. For example, the improvement information generation unit 46 may generate, as the improvement information, information indicating a recommendation to engage in learning.

For example, when the mental state of the user is classified as the stressed state by the autonomic nervous system acquisition unit 42, the prediction information generation unit 45 may generate, as the mental information, information indicating that the user tends to feel stressed. In this case, the improvement information generation unit 46 may generate, as the improvement information, information indicating a recommendation to practice meditation.

When the prediction information generation unit 45 generates, as the prediction information, information indicating that the mood of the user is predicted to tend to become depressed, for example, the improvement information generation unit 46 may generate, as the improvement information, at least one of information indicating contents that improve the depressed mood (for example, movies, music, games, or books) and information regarding travel (for example, travel destinations, accommodations, tourist attractions, and the like).

The lifestyle improvement system 1 may be used, for example, in at least one of financial institutions, medical institutions, automobiles, home appliances, beauty product, clothing and food stores, e-commerce, and cram schools. The lifestyle improvement system 1 may be used to provide products and services suited to each user, based on at least one of the past sleep information and the autonomic nervous system information of the user.

### Reference Signs List

1: lifestyle improvement system, 2, 14: core material, 3: cover fabric, 10a: mattress, 10b: cushion, 11: sensor unit, 12: sensor sheet, 13: cushion sensor, 13a: seating portion sensor, 13b: sacrum support portion sensor, 13c: thigh support portion sensor, 15: seating portion, 15a: buttock support portion, 15b: thigh support portion, 16: lumbar support portion, 16a: general portion, 16b: sacrum support portion, 21: upper surface, 22: lower surface, 23: side surface, 31: front fabric, 32: back fabric, 33: opening and closing member, 34: slide member, 35: opening and closing portion, 40: lifestyle improvement application, 41: sleep state acquisition unit, 42: autonomic nervous system acquisition unit, 43: storage unit, 44: device control unit, 45: prediction information generation unit, 46: improvement information generation unit, 47: arrhythmia detection unit, 80: input screen, 81: sleep evaluation input portion, 82, 85: output screen, 83: prediction information display portion, 84: improvement information display portion, 86: subjective information display portion, 87: objective information display portion, 88: feedback display portion, 101: information terminal, 102: lifestyle improvement server, 103: website, 104: device, 105: device server, A1: first direction, A2: second direction, A3: third direction, D1: longitudinal direction, D2: lateral direction, D3: thickness direction.

## Claims

1. A lifestyle improvement system for improving a lifestyle of a user, comprising:
a sensor unit that acquires at least one of respiratory information that is information indicating a respiration of the user, body movement information that is information indicating a body movement of the user, and heartbeat information that is information indicating a heartbeat of the user;
a sleep state acquisition unit that acquires sleep state information, which is information indicating a sleep state of the user, from at least one of the respiratory information, the body movement information, and the heartbeat information;
an autonomic nervous system acquisition unit that acquires autonomic nervous system information, which is information indicating a state of an autonomic nervous system of the user, from the heartbeat information;
a device control unit that controls an operation of a device constituting an environment surrounding the user, based on at least one of past sleep information, which is past sleep state information of the user stored in advance, and the autonomic nervous system information;
a prediction information generation unit that generates prediction information, which is information indicating a predicted state of the user while awake, from at least one of the past sleep information and the autonomic nervous system information; and
an improvement information generation unit that generates improvement information, which is information for improving the lifestyle of the user, from the prediction information.

2. The lifestyle improvement system according to claim 1,
wherein the sensor unit includes a cushion sensor attached to a cushion on which the user is seated.

3. The lifestyle improvement system according to claim 1 or 2,
wherein the prediction information includes mental information that is information indicating a mental state of the user, physical condition information that is information indicating a physical condition of the user, and brain information that is information indicating a state of a brain of the user.

4. The lifestyle improvement system according to claim 3,
wherein the physical condition information includes skin information indicating a state of a skin of the user.

5. The lifestyle improvement system according to claim 1 or 2,
wherein the improvement information generation unit generates the improvement information from subjective information that is information indicating a subjective evaluation of the user.

6. The lifestyle improvement system according to claim 1 or 2,
wherein the improvement information includes information indicating a type of clothing recommended for the user.

7. The lifestyle improvement system according to claim 1 or 2,
wherein the improvement information includes information indicating a type of food recommended for the user.

8. The lifestyle improvement system according to claim 1 or 2,
wherein the improvement information includes information indicating a type of cosmetics recommended for the user.

9. The lifestyle improvement system according to claim 1 or 2,
wherein the device includes a pillow used by the user for sleeping, and
the device control unit controls an angle of a placement surface of the pillow, on which a head of the user is placed, with respect to a horizontal plane.

10. The lifestyle improvement system according to claim 1 or 2, further comprising:
an arrhythmia detection unit that detects a presence or absence of arrhythmia in the user from the heartbeat information acquired by the sensor unit.

11. A lifestyle improvement system for improving a lifestyle of a user, comprising:
a sensor unit that acquires heartbeat information that is information indicating a heartbeat of the user;
an autonomic nervous system acquisition unit that acquires autonomic nervous system information, which is information indicating a state of an autonomic nervous system of the user, from the heartbeat information;
a prediction information generation unit that generates prediction information, which is information indicating a predicted state of the user while awake, from the autonomic nervous system information; and
an improvement information generation unit that generates improvement information, which is information for improving the lifestyle of the user, from the prediction information.
